(19) **Europäisches Patentamt**
**European Patent Office**
**Office européen des brevets**

(11) **EP 4 246 137 A1**

(12) **EUROPEAN PATENT APPLICATION**

(43) Date of publication:
**20.09.2023 Bulletin 2023/38**

(21) Application number: **22161857.2**

(22) Date of filing: **14.03.2022**

(51) International Patent Classification (IPC):
***G01N 27/12*** (2006.01)   ***G01N 33/00*** (2006.01)

(52) Cooperative Patent Classification (CPC):
**G01N 27/124; G01N 33/0034**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Infineon Technologies AG**
**85579 Neubiberg (DE)**

(72) Inventors:
• **BREUER, Werner**
**93161 Sinzing-Viehhausen (DE)**
• **CARBONELLI, Cecilia**
**81477 München (DE)**

• **MITTERMAIER, Simon**
**85659 Forstern (DE)**
• **ROTH, Alexandra Marina**
**92318 Neumarkt (DE)**
• **ZHAO, Jianyu**
**81829 München (DE)**
• **ZOEPFL, Alexander**
**93049 Regensburg (DE)**

(74) Representative: **Hersina, Günter et al**
**Schoppe, Zimmermann, Stöckeler**
**Zinkler, Schenk & Partner mbB**
**Patentanwälte**
**Radlkoferstrasse 2**
**81373 München (DE)**

(54) **GAS SENSING DEVICE FOR SENSING ONE OR MORE GASES IN A MIXTURE OF GASES**

(57)    The gas sensing device comprising:
one or more chemo-resistive gas sensors, wherein each of the gas sensors is configured for generating signals corresponding to concentrations of the one or more gases in the mixture of gases;
a heating arrangement configured in such way that the gas sensors are heated according to a periodic temperature profile;
a preprocessing processor configured for receiving the signals from each of the gas sensors and for preprocessing the received signals in order to generate a preprocessed signal sample for each of the gas sensors for each period of the periodic temperature profile;
a feature extraction processor configured for receiving the preprocessed signal samples and for extracting for each of the periods a set of feature values from the preprocessed signal samples received for the respective period, wherein each of the sets of feature values comprises one or more time domain feature values, which are based on time domain characteristics of one of the preprocessed signal samples received for the respective period, and one or more frequency domain feature values, which are based on frequency domain characteristics of one of the preprocessed signal samples received for the respective period;
a gas concentration processor configured for receiving the sets of feature values,
and for creating for each of the sets of feature values a

sensing result SR for each of the gases, wherein the gas concentration processor comprises a trained model based algorithm processor and one or more trained models for the trained model based algorithm processor, wherein at least a portion of the respective set of feature values is fed to inputs of the trained model based algorithm processor using one of the one or more trained models, wherein the sensing results are based on output values at outputs of the trained model based algorithm processor.

Fig. 1

EP 4 246 137 A1

## Description

Technical Field

Embodiments relate to a gas sensing device for sensing one or more gases in a mixture of gases. Further embodiments relate to a method for operating such gas sensing device. More particular, the disclosure deals with the estimation of gas concentrations through the use of chemo-resistive gas sensors.

Background of the Invention

Literature on chemo-resistive gas sensors is generally limited to a simple model for proof of sensor functionality or costly data acquisition methodologies using geographically distributed sensor systems with impractical implementations [1, 2].
Temperature cycling has been used in the context of MOX sensors to be able to differentiate different gases with a single sensor [3], [4], [5]. However, related studies were quite preliminary and no appropriate feature extraction and gas separation method was provided. In addition, MOX temperature levels are relatively high causing large heat dissipation and energy consumption and making miniaturization quite complex.

Summary of the Invention

Disclosed is a gas sensing device for sensing one or more gases in a mixture of gases is provided. The gas sensing device comprises:

one or more chemo-resistive gas sensors, wherein each of the gas sensors is configured for generating signals corresponding to concentrations of the one or more gases in the mixture of gases;

a heating arrangement configured in such way that the gas sensors are heated according to a periodic temperature profile;

a preprocessing processor configured for receiving the signals from each of the gas sensors and for preprocessing the received signals in order to generate a preprocessed signal sample for each of the gas sensors for each period of the periodic temperature profile;

a feature extraction processor configured for receiving the preprocessed signal samples and for extracting for each of the periods a set of feature values from the preprocessed signal samples received for the respective period, wherein each of the sets of feature values comprises one or more time domain feature values, which are based on time domain characteristics of one of the preprocessed signal samples received for the respective period, and one or more frequency domain feature values, which are based on frequency domain characteristics of one of the preprocessed signal samples received for the respective period;

a gas concentration processor configured for receiving the sets of feature values, and for creating for each of the sets of feature values a sensing result for each of the gases, wherein the gas concentration processor comprises a trained model based algorithm processor and one or more trained models for the trained model based algorithm processor, wherein at least a portion of the respective set of feature values is fed to inputs of the trained model based algorithm processor using one of the one or more trained models, wherein the sensing results are based on output values at outputs of the trained model based algorithm processor.

The one or more chemo-resistive gas sensors may be graphene gas sensors or reduced graphene gas sensors, where the base material is functionalized with specific chemicals, e.g. with platinum (Pt), or manganese dioxide (MnO2), so that each of the gas sensors is sensitive for gases, e.g. for nitrogen dioxide (NO2), ozone (O3) or carbon monoxide (CO). In doing so, the interaction between graphene sheets and absorbed gas analytes influences the electronic structure of the material depending on the mixture of gases, resulting in altered charge carrier concentration and changed electrical conductance.
In case of multi-gas sensing a multi-gas sensor array comprising a plurality of chemo-resistive gas sensors having dissimilar selectivity may be used. Due to the different sensitivity towards various gas molecules, resistances of the gas sensors change in disparate patterns, making it possible to analyze complicated gas mixtures with one single sensor array.
The signals are typically electrical signals, which refer to electrical properties of the sensing material of one of the gas sensors, which depend on the gases contained in the mixture of gases. The electrical properties may include the electrical resistance or the electrical conductivity of the sensing material. The signals may be time-discrete signals or continuous-time signals.
Each of the gas sensors may be heated by one or more heat sources of the heating arrangement. The heat sources may be electrically powered resistive heat sources or radiators emitting light, in particular with ultra violet light. Each of the one or more heat sources is controlled according to one or more periodic temperature profiles during operational phases.
Each of the periodic temperature profiles modulates a temperature of one or more of the gas sensors between a maximum temperature and a minimum temperature. The maximum temperature may be, for example, set to a value between 150°C and 300°C, whereas the minimum temperature may be, for example, set to a value between 50°C and 200°C. Different temperatures may be used for different of the gas sensors at the same time. However, the period length, which is the time of one full oscillation, is the same for all of the gas sensors, which facilitates the analyses of the signals.

The term processor refers to an electronic device configured for specific task. A processor may comprise hardware or a combination of hardware and software. Different processors may share hardware components and/or software components.

The pre-processing processor is configured for suppressing and/or compensating of artifacts in the signal samples and/or noise in the signal samples and/or invalid signal samples due to malfunctioning gas sensors and/or errors in the signal samples due to drifts of the gas sensors in order to produce more reliable preprocessed signal samples.

Each of the preprocessed signal samples is a sequence consisting of time-discrete signal values, wherein the signal values depend on the signal from one of the gas sensors. Each of the preprocessed signal samples represents a time interval of the respective signal, which is equivalent to the duration of a period of the periodic temperature profile.

The feature extraction processor is configured for receiving the preprocessed signal samples and for extracting for each of the periods a set of feature values from the preprocessed signal samples received for the respective period. This means, that a set of feature values for a specific period may depend on a plurality of preprocessed signal samples, which originate from different gas sensors.

Furthermore, the feature extraction processor is configured in such way that each of the sets of feature values comprises one or more time domain feature values, which are based on time domain characteristics of one of the preprocessed signal samples received for the respective period, and one or more frequency domain feature values, which are based on frequency domain characteristics of one of the preprocessed signal samples received for the respective period. Some examples for time domain feature values and some examples frequency domain feature values are given below.

The gas concentration processor is configured for creating for each of the gases a sensing result. The sensing results may be alphanumeric terms, for example alphanumeric terms on a scale from "high" to "low". In particular, the terms of an air quality index system, for example terms of the European air quality index, may be used for outputting the sensing results. In other embodiments, the sensing results may be physical quantities such as "4% by volume".

A trained model based algorithm processor is a processor, which is capable of machine learning. The machine learning is done in a preoperational training phase in which trained models are developed by comparing actual output values of the trained model based algorithm stage with desired output values of the trained model based algorithm stage for defined inputs of the trained model based algorithm stage. The trained models have a predefined structure, wherein a parametrization of the predefined structure is done during the training phase. The trained models comprise the learned content after the training phase is finished. In an operational phase for producing processing results one or more of the trained models from the training phase are used to process their input data.

In the training phase the plurality of trained models can be established and afterwards stored at the gas sensing device. The trained models may differ in the structures and/or the parameters. During the operation phase the most appropriate trained model may be selected depending on the on the specific use-case.

The disclosed gas sensing device overcomes drawbacks of prior art sensors such as cross-sensitivities to other gases and the drifting sensor response due to aging and other environmental effects by controlling the heating in a periodic motion rather than in discrete states, and by extracting sets of feature values which refer to time domain characteristics as well as to frequency domain characteristics of the preprocessed signal samples. The periodic temperature modulation still allows for phases with higher temperatures for faster sensor recovery. Meanwhile, it also introduces dynamics to the signal response that can be harnessed by astute signal processing in time domain and in frequency domain.

The gas sensing device can reflect real world scenarios, where, for example, gas mixtures are present which are causing cross-sensitivities in the sensor responses. In particular, the proposed gas sensing device is capable of distinguishing between different gases with reasonable accuracy, in particular between NO2 and O3, which are difficult to distinguish with chemo-resistive sensors.

The gas sensing device according to the disclosure addresses the intrinsic instability of chemo-resistive gas sensors. It uses robust algorithms and detection mechanisms, which can cope with calibration inaccuracies, drifts and other similar effects reliably and over a wide operating range. Moreover, the gas sensing device only takes a short time for reaching a stable response level.

The proposed gas sensing device provides an end to end solution for multi-gas adsorption sensors which is versatile, widely-applicable to multiple applications and uses cases (outdoor, indoor, health check, etc.) and can be embedded in a smart portable device. Specifically, an algorithm is used that works on continuous sensor readings, makes use of the transient information in the sensor responses and exhibits low complexity and limited memory requirements.

According to embodiments of the disclosure the preprocessing processor comprises a baseline normalization processor configured for normalizing the signals received from the gas sensors. Baseline normalization is the transformation of a signal sample of one of the gas sensors into a relative resistance or conductivity change with respect to sensor response to a reference analyte, wherein such sensor response is called a baseline. Synthetic air is a very common baseline as it is easily applicable and realistic in a real world scenario. The purpose of a baseline is to potentially create a more stable and reproducible sensing result by removing some of the drift caused by long term gas exposure and ageing of the sensor.

According to embodiments of the disclosure the preprocessing processor comprises a sine period extraction processor configured for receiving a control signal from the heating arrangement and for extracting a starting time and an end time for one of the periods from the control signal. These features ensure that the subsequent signal processing is synchronized with the control of the heating arrangement.

According to embodiments of the disclosure the feature extraction processor comprises a slope calculation processor configured for calculating for each of the periods a slope for each of the preprocessed signal samples received for the respective period, wherein the feature extraction processor is configured for using the slope of one of the periods as one of the time domain feature values of the set of feature values of the respective period. The slope of a preprocessed signal sample is the difference between the value of the preprocessed signal sample at the end time of the preprocessed signal sample and the value of the preprocessed signal sample at the start time of the preprocessed signal sample. The slope may be used as a time domain feature value and for flattening the preprocessed signal sample by subtracting the slope proportionally to the time from the preprocessed signal sample.

According to embodiments of the disclosure the feature extraction processor comprises a sensitivity calculation processor configured for calculating for each of the periods a sensitivity for each of the preprocessed signal samples received for the respective period, wherein the feature extraction processor is configured for using the sensitivity of one of the periods as one of the time domain feature values of the set of feature values of the respective period. The sensitivity of a sensor is in general the slope dy/dx assuming a linear characteristic, where dy is the change of an output and where dx is the change of an input. The sensitivity may be calculated from the flattened preprocessed signal sample by calculating a mean value of the flattened preprocessed signal sample.

According to embodiments of the disclosure the feature extraction processor comprises a time-to-frequency converting processor configured for converting each of the preprocessed signal samples from a time domain into a frequency domain in order to calculate a spectrum for each of the preprocessed signal samples, wherein the frequency domain feature values for the respective preprocessed signal sample are calculated from the respective spectrum. The time-to-frequency converting processor may be configured for subtracting the mean value from the flattened preprocessed signal sample in order to remove a DC-offset in order to produce filtered preprocessed signal sample. The time-to-frequency converting processor may also be configured for applying a fast Fourier transform algorithm to the filtered preprocessed signal sample in order to calculate the spectrum.

According to embodiments of the disclosure the feature extraction processor comprises an amplitude calculation processor configured for calculating for each of the periods at least one amplitude of the spectrum of each of the preprocessed signal samples received for the respective period, wherein the feature extraction processor is configured for using at least one of the amplitudes calculated for one of the periods as one of the frequency domain feature values of the set of feature values of the respective period. Each of the amplitudes of a specific spectrum may refer to one bin of the spectrum. In some embodiments, only the amplitude of the fundamental frequency is used. In other embodiments, the amplitude of the fundamental frequency and/or amplitudes of one or more harmonics are used.

According to embodiments of the disclosure the feature extraction processor comprises a phase calculation processor configured for calculating for each of the periods at least one phase of the spectrum of each of the preprocessed signal samples received for the respective period, wherein the feature extraction processor is configured for using at least one of the phases calculated for one of the periods as one of the frequency domain feature values of the set of feature values of the respective period. Each of the phases of a specific spectrum may refer to one bin of the spectrum. In some embodiments, only the phase of the fundamental frequency is used. In other embodiments, the phase of the fundamental frequency and/or phases of one or more harmonics are used.

According to embodiments of the disclosure the feature extraction processor comprises a total harmonic distortion calculation processor configured for calculating for each of the periods at least one total harmonic distortion of the spectrum of each of the preprocessed signal samples received for the respective period, wherein the feature extraction processor is configured for using at least one of the total harmonic distortions calculated for one of the periods as one of the frequency domain feature values of the set of feature values of the respective period. The total harmonic distortion may be calculated as the ratio of the square root of the quadratic sum of the amplitude of a specified number of higher order harmonics to the fundamental frequency.

According to embodiments of the disclosure the preprocessing processor comprises a relative resistance extracting processor configured for extracting relative resistance changes of the gas sensors from the signals received from the gas sensors, wherein the preprocessing processor is configured for using the relative resistance changes for generating the preprocessed signal samples.

According to embodiments of the disclosure the preprocessing processor comprises a relative conductance extracting processor configured for extracting relative conductance changes of the gas sensors from the signals received from the gas sensors, wherein the preprocessing processor is configured for using the relative conductance changes for generating the preprocessed signal samples.

The choice, whether the relative resistance changes or the relative conductance changes are used as a basis for subsequent signal processing is made based on which shows the more linear behavior over time (linear resistance drift vs. linear conductance drift) in order to achieve the best overall performance and stability.

According to embodiments of the disclosure the gas sensing device comprises

a drift calculation processor configured for calculating for each of the sensors a drift of the respective sensor signal, and

a feature selection processor configured for selecting for each of the sensors, based

on the drift of the respective sensor, which of the feature values of one of the sets of feature values are fed to the inputs of the trained model based algorithm processor.

By these features, a degradation of the overall performance due to drift may be minimized.

According to embodiments of the disclosure, the periodic temperature profile is a sinusoidal temperature profile. Using a sinusoidal temperature profile facilitates the subsequent preprocessing and feature extraction and provides most reliable sensing results. However, other periodic temperature profiles such as multi-sinusoidal temperature profiles, rectangular temperature profiles etc. are possible, as long as they are periodic

In a further aspect of the disclosure, a method for operating a gas sensing device for sensing one or more gases in a mixture of gases, which comprises one or more chemo-resistive gas sensors is disclosed. The method comprises the steps of:

using each of the gas sensors for generating signals corresponding to concentrations of the one or more gases in the mixture of gases;

using a heating arrangement in such way that the gas sensors are heated according to a periodic temperature profile;

using a preprocessing processor for receiving the signals from each of the gas sensors and for preprocessing the received signals in order to generate a preprocessed signal sample for each of the gas sensors for each period of the periodic temperature profile;

using a feature extraction processor for receiving the preprocessed signal samples and for extracting for each of the periods a set of feature values from the preprocessed signal samples received for the respective period, wherein each of the sets of feature values comprises one or more time domain feature values, which are based on time domain characteristics of one of the preprocessed signal samples received for the respective period, and one or more frequency domain feature values, which are based on frequency domain characteristics of one of the preprocessed signal samples received for the respective period;

using a gas concentration processor for receiving the sets of feature values, and for creating for each of the sets of feature values a sensing result for each of the gases, wherein the gas concentration processor comprises a trained model based algorithm processor and at least one trained model for the trained model based algorithm processor, wherein at least a portion of the respective set of feature values is fed to inputs of the trained model based algorithm processor, wherein the sensing results are based on output values at outputs of the trained model based algorithm processor.

Preferred embodiments of the invention are subsequently discussed with respect to the accompanying drawings, in which:

Figure 1    shows a schematic view of a first exemplary embodiment of a gas sensing device according to the disclosure, which comprises four chemo-resistive gas sensors;

Figure 2    illustrates an exemplary period of a periodic temperature profile provided by a heating arrangement and a signal provided by a chemoresistive gas sensor heated according to the periodic temperature profile;

Figure 3    shows a schematic view of a second exemplary embodiment of a gas sensing device according to the disclosure, which comprises four chemo-resistive gas sensors;

Figure 4    illustrates an exemplary plot of an exemplary relative estimation error as a function of the drift rate for different sets of feature values;

Figure 5    shows an exemplary graphene multi-gas sensor array according to the disclosure;

Figure 6    illustrates an exemplary implementation of the feature extraction for a periodic sensor signal;

Figure 7     illustrates an exemplary preprocessed signal sample and a spectrum derived therefrom;

Figure 8     illustrates exemplary training steps for a trained model using linear discriminant analysis;

Figure 9     illustrates exemplary plots of exemplary time domain feature values and of exemplary frequency domain feature values for a specific gas concentration profile; and

Figure 10    illustrates an exemplary plot of an exemplary relative estimation error and of an exemplary R2 score for time domain feature values and for frequency domain feature values as a function of time.

Equal or equivalent elements or elements with equal or equivalent functionality are denoted in the following description by equal or equivalent reference numerals.

In the following description, a plurality of details is set forth to provide a more thorough explanation of embodiments of the present disclosure. However, it will be apparent to those skilled in the art that embodiments of the present disclosure may be practiced without these specific details. In other instances, well-known structures and devices are shown in block diagram form rather than in detail in order to avoid obscuring embodiments of the present disclosure. In addition, features of the different embodiments described hereinafter may be combined with each other, unless specifically noted otherwise.

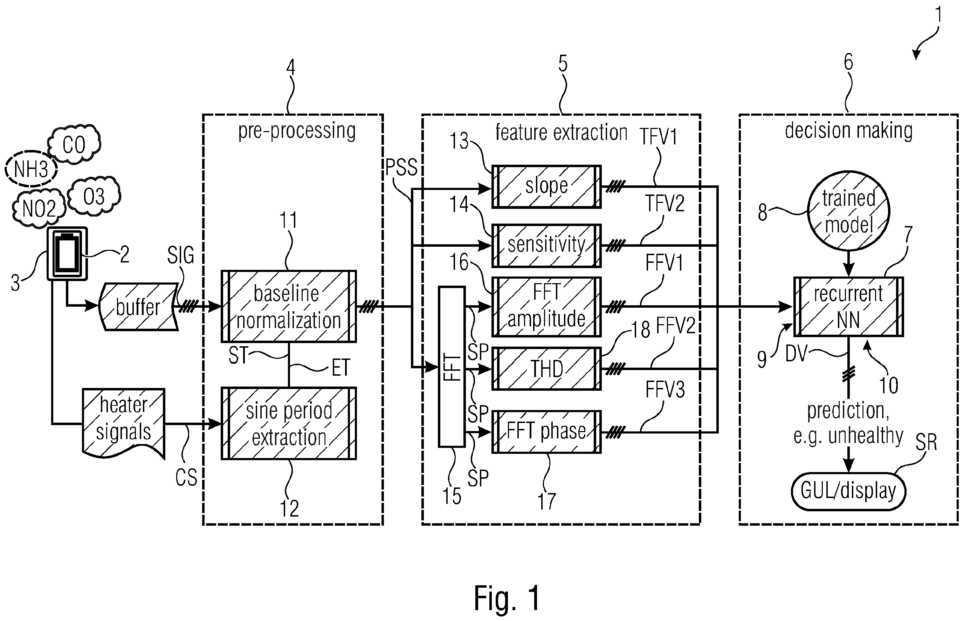

Figure 1 shows a schematic view of a first exemplary embodiment of a gas sensing device 1 according to the disclosure, which comprises four chemo-resistive gas sensors 2.

The gas sensing device 1 is configured for sensing one or more gases in a mixture of gases. The gas sensing device 1 comprises:

one or more chemo-resistive gas sensors 2, wherein each of the gas sensors 2 is configured for generating signals SIG corresponding to concentrations of the one or more gases in the mixture of gases;

a heating arrangement 3 configured in such way that the gas sensors 2 are heated according to a periodic temperature profile STP;

a preprocessing processor 4 configured for receiving the signals SIG from each of the gas sensors 2 and for preprocessing the received signals SIG in order to generate a preprocessed signal sample PSS for each of the gas sensors 2 for each period PER of the periodic temperature profile STP;

a feature extraction processor 5 configured for receiving the preprocessed signal samples PSS and for extracting for each of the periods PER a set of feature values TFV, FFV from the preprocessed signal samples PSS received for the respective period PER, wherein each of the sets of feature values TFV, FFV comprises one or more time domain feature values TFV, which are based on time domain characteristics of one of the preprocessed signal samples PSS received for the respective period PER, and one or more frequency domain feature values FFV, which are based on frequency domain characteristics of one of the preprocessed signal samples PSS received for the respective period PER;

a gas concentration processor 6 configured for receiving the sets of feature values TFV, FFV, and for creating for each of the sets of feature values TFV, FFV a sensing result SR for each of the gases, wherein the gas concentration processor 6 comprises a trained model based algorithm processor 7 and one or more trained models 8 for the trained model based algorithm processor 7, wherein at least a portion of the respective set of feature values TFV, FFV is fed to inputs 9 of the trained model based algorithm processor 7 using one of the one or more trained models 8, wherein the sensing results SR are based on output values OV at outputs 10 of the trained model based algorithm processor 7.

According to embodiments of the disclosure the preprocessing processor 4 comprises a baseline normalization processor 11 configured for normalizing the signals SIG received from the gas sensors 2.

According to embodiments of the disclosure the preprocessing processor 4 comprises a sine period extraction processor 12 configured for receiving a control signal CS from the heating arrangement 3 and for extracting a starting time ST and an end time ET for one of the periods PER from the control signal CS.

According to embodiments of the disclosure the feature extraction processor 5 comprises a slope calculation processor 13 configured for calculating for each of the periods PER a slope for each of the preprocessed signal samples PSS received for the respective period PER, wherein the feature extraction processor 5 is configured for using the slope of one of the periods PER as one of the time domain feature values TFV of the set of feature values TFV, FFV of the respective period PER.

According to embodiments of the disclosure the feature extraction processor 5 comprises a sensitivity calculation processor 14 configured for calculating for each of the periods PER a sensitivity for each of the preprocessed signal samples PSS received for the respective period PER, wherein the feature extraction processor 5

is configured for using the sensitivity of one of the periods PER as one of the time domain feature values TFV of the set of feature values TFV, FFV of the respective period PER.

According to embodiments of the disclosure the feature extraction processor 5 comprises a time-to-frequency converting processor 15 configured for converting each of the preprocessed signal samples PSS from a time domain into a frequency domain in order to calculate a spectrum SP for each of the preprocessed signal samples PSS, wherein the frequency domain feature values FFV for the respective preprocessed signal sample PSS are calculated from the respective spectrum SP.

According to embodiments of the disclosure the feature extraction processor 5 comprises an amplitude calculation processor 16 configured for calculating for each of the periods PER at least one amplitude of the spectrum SP of each of the preprocessed signal samples PSS received for the respective period PER, wherein the feature extraction processor 5 is configured for using at least one of the amplitudes calculated for one of the periods PER as one of the frequency domain feature values FFV of the set of feature values TFV, FFV of the respective period PER.

According to embodiments of the disclosure the feature extraction processor 5 comprises a phase calculation processor 17 configured for calculating for each of the periods PER at least one phase of the spectrum SP of each of the preprocessed signal samples PSS received for the respective period PER, wherein the feature extraction processor 5 is configured for using at least one of the phases calculated for one of the periods PER as one of the frequency domain feature values FFV of the set of feature values TFV, FFV of the respective period PER.

According to embodiments of the disclosure the feature extraction processor 5 comprises a total harmonic distortion calculation processor 18 configured for calculating for each of the periods PER at least one total harmonic distortion of the spectrum SP of each of the preprocessed signal samples PSS received for the respective period PER, wherein the feature extraction processor 5 is configured for using at least one of the total harmonic distortions calculated for one of the periods PER as one of the frequency domain feature values FFV of the set of feature values TFV, FFV of the respective period PER.

In a further aspect, the disclosure refers to a method for operating a gas sensing device 1 for sensing one or more gases in a mixture of gases, wherein the gas sensing device 1 comprises one or more chemoresistive gas sensors 2, wherein the method comprises the steps of:

  using each of the gas sensors 2 for generating signals SIG corresponding to concentrations of the one or more gases in the mixture of gases;

  using a heating arrangement 3 in such way that the gas sensors 2 are heated according to a periodic temperature profile STP;

  using a preprocessing processor 4 for receiving the signals SIG from each of the gas sensors 2 and for preprocessing the received signals SIG in order to generate a preprocessed signal sample PSS for each of the gas sensors 2 for each period PER of the periodic temperature profile STP;

  using a feature extraction processor 5 for receiving the preprocessed signal samples PSS and for extracting for each of the periods PER a set of feature values TFV, FFV from the preprocessed signal samples PSS received for the respective period PER, wherein each of the sets of feature values TFV, FFV comprises one or more time domain feature values TFV, which are based on time domain characteristics of one of the preprocessed signal samples PSS received for the respective period PER,

  and one or more frequency domain feature values FFV, which are based on frequency domain characteristics of one of the preprocessed signal samples PSS received for the respective period PER;

  using a gas concentration processor 6 for receiving the sets of feature values TFV, FFV, and for creating for each of the sets of feature values TFV, FFV a sensing result SR for each of the gases, wherein the gas concentration processor 6 comprises a trained model based algorithm processor 7 and at least one trained model 8 for the trained model based algorithm processor 7, wherein at least a portion of the respective set of feature values TFV, FFV is fed to inputs 9 of the trained model based algorithm processor 7, wherein the sensing results SR are based on output values OV at outputs 10 of the trained model based algorithm processor 7.

The overall algorithm flow starts with collecting the measured signals SIG, which may be resistance data of the respective sensor 2. An inversion of the resistance signals is performed if they appear to have a more linear conductance drift than resistance drift. In a pre-processing processor 4, the signals SIG are normalized with a baseline and individual sine periods PER are confined for the feature extraction processor 5. The extracted features TFV, FFV are then used as input to a trained model based algorithm processor 7 that performs the actual prediction of gas concentrations.

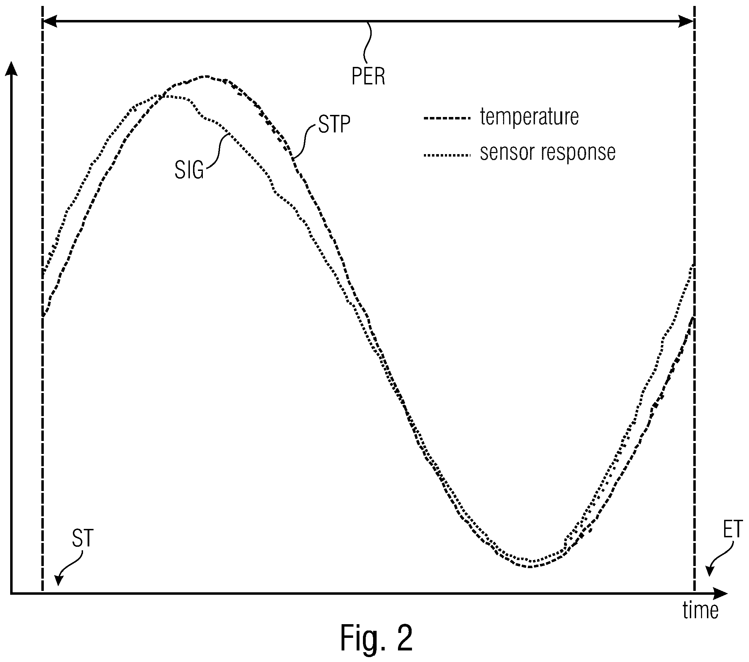

Figure 2 illustrates an exemplary period of a periodic temperature profile STP provided by a heating arrangement 3 and a signal SIG provided by a chemo-resistive gas sensor 2 heated according to the periodic temperature profile STP.

According to embodiments of the disclosure the periodic temperature profile STP is a sinusoidal temperature profile STP.

As shown in Figure 2, the signal SIG, which refers to the conductance of the sensor 2, follows the sinusoidal temperature profile STP to some extend but is distorted in comparison. This distortion is more pronounced when the sensor 2 comes in to contact with higher gas concentrations and is not present if there is no sensor reactive gas present. The added distortion increases the information content in the signal SIG, which can be exploited to improve gas detection. By transforming the signal SIG, for example via Fourier transformation, into the frequency domain, frequency domain feature values FFV that capture this information can be extracted, e.g., the total harmonic distortion (THD) and the phase shift of the fundamental frequency component.

Frequency domain feature values FFV extracted in the frequency domain based on the dynamic of the signal SIG offer various advantages. One of them is the observation that in the event of changing gas concentrations, the impact on the dynamic of the signal SIG is immediate whereas the resistance change shows some hysteresis. The frequency domain features values FFV therefore offer a quick response to changes of the signal SIG. Another advantage is that the amount of distortion is independent from baseline changes (where by baselines the initial value of the sensor resistance in the presence of clean air is meant) and thus the corresponding frequency domain feature values FFV have the attribute of added robustness against drift in the signal SIG of the sensor 2. Furthermore, under a certain mode of operation (max. temperature, period length), it was observed that the distortion of the signal SIG is only experienced for ozone (O3) whereas it is not visible for other sensor reactive gases like nitrogen dioxide (NO2). This in turn yields frequency domain feature values FFV that are highly selective to ozone and show less cross- sensitivity to other gases, which can be efficiently exploited by judiciously designed signal processing.

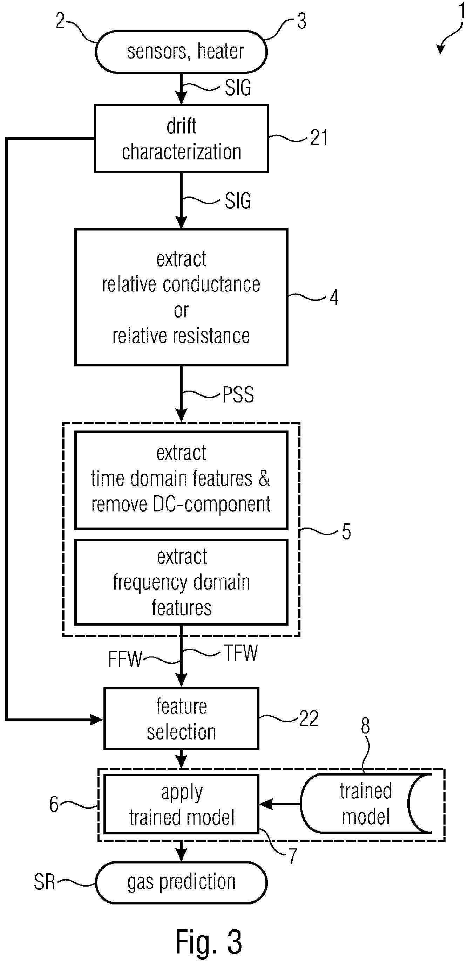

Figure 3 shows a schematic view of a second exemplary embodiment of a gas sensing device 1 according to the disclosure, which comprises four chemoresistive gas sensors 2.

According to embodiments of the disclosure the preprocessing processor 4 comprises a relative resistance extracting processor 19 configured for extracting relative resistance changes of the gas sensors 2 from the signals SIG received from the gas sensors 2, wherein the preprocessing processor 4 is configured for using the relative resistance changes for generating the preprocessed signal samples PSS.

According to embodiments of the disclosure the preprocessing processor 4 comprises a relative conductance extracting processor 20 configured for extracting relative conductance changes of the gas sensors 2 from the signals SIG received from the gas sensors 2, wherein the preprocessing processor 4 is configured for using the relative conductance changes for generating the preprocessed signal samples PSS.

According to embodiments of the disclosure the gas sensing device 2 comprises

a drift calculation processor 21 configured for calculating for each of the sensors 2 a drift of the respective sensor signal SIG, and

a feature selection processor 22 configured for selecting for each of the sensors 2, based on the drift of the respective sensor 2, which of the feature values TFV, FFV of one of the sets of feature values TFV, FFV are fed to the inputs 9 of the trained model based algorithm processor 7.

Figure 3 describes an embodiment of the gas sensing device 1 with a flow diagram. Starting with the signal SIG provided by the sensor 2, the resistance or the conductance of the sensor 2 is extracted. The latter provides a better visual alignment with the heating temperature, as seen in Figure 2, but mainly the choice whether to use resistance or conductance as basis for further processing steps should be made based on which shows the more linear behavior over time (linear resistance drift vs. linear conductance drift) for the best overall performance and stability.

The following feature extraction processor 5 works both for resistance and for conductance. After the feature extraction processor 5, feature values TFV, FFV may be selected based on the application and their drift behavior to be fed to inputs 9 of the trained model based algorithm processor 7. If the sensor 2 is stable over time, all available features values TFV, FW may be used to provide the most information to the trained model 8 which results in the best possible performance. If the sensor 2 experiences strong drift, which results in a large baseline offset, the information of the time domain feature values TFV degenerates. Consequently, only frequency domain feature values FFV may be used to have a stable and robust performance even under such conditions. For each scenario an individual trained model 8 may be used that is specifically trained for the chosen set of feature values TFV, FFV.

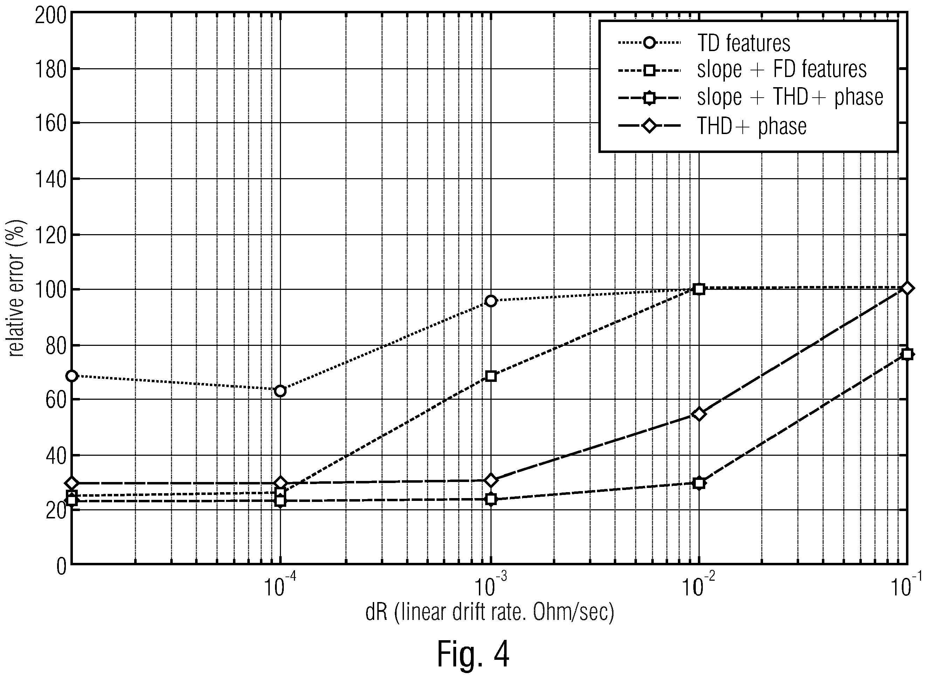

Figure 4 illustrates an exemplary plot of an exemplary relative estimation error as a function of the drift rate for different sets of feature values TFV, FFV. Figure 4 shows an example of how different sets of feature values TFV, FFV perform differently in the presence of a linear upward resistance drift. Depending on the drift rate (dR, in Ohm per second) different sets of feature values TFV, FFV may be selected as a tradeoff between performance, complexity and generalization capabilities to other gases.

The task of characterizing the drift can be seen as part of the overall design procedure for the gas sensing device 1. Here accelerated stress tests may be carried out to describe the long term behavior of the gas sensing device 1 and thus its ageing properties. Alternatively, the gas sensing device 1 can be operated in the field and observed over a long period of time (e.g. months) until its drift properties are fully described and correlated to other environmental factors. Last but not least, also the observation of the own data can be leveraged to understand if the drift is linear or not, for example by comparing long term and short term (few days) derivative trends or by correlating drifting and non-drifting features.

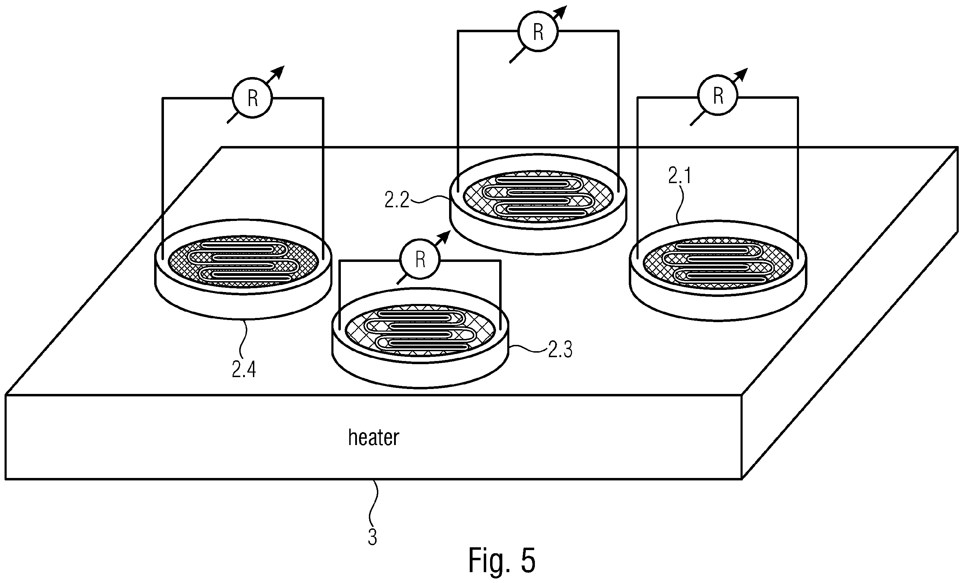

Figure 5 shows an exemplary graphene multigas sensor array according to the disclosure. Each sensor 2.1, 2.2, 2.3 and 2.4 in the array is heated by a heat source of a heating arrangement 3, whose temperature is being controlled according to a periodic temperature profile. In other embodiments, the sensors 2.1, 2.2, 2.3 and 2.4 in the array are heated by a plurality of heat sources. For example, each of the sensors 2.1, 2.2, 2.3 and 2.4 could be heated individually by one heat source of the plurality of the heat sources.

The sensors 2.1, 2.2, 2.3 and 2.4 form a multigas sensor array, where a base material consisting of graphene is functionalized with different chemicals (e.g. Pd, Pt, and MnO2) for dissimilar selectivity. The interaction between graphene sheets and absorbed gas analytes would influence the electronic structure of the material, resulting in altered charge carrier concentrations and changed electrical conductance or resistance respectively. Meanwhile, due to different sensitivity towards various gas molecules resistances of the sensors 2.1, 2.2, 2.3 and 2.4 also change in disparate patterns, making it possible to analyze complicated gas mixtures with one single sensor array.

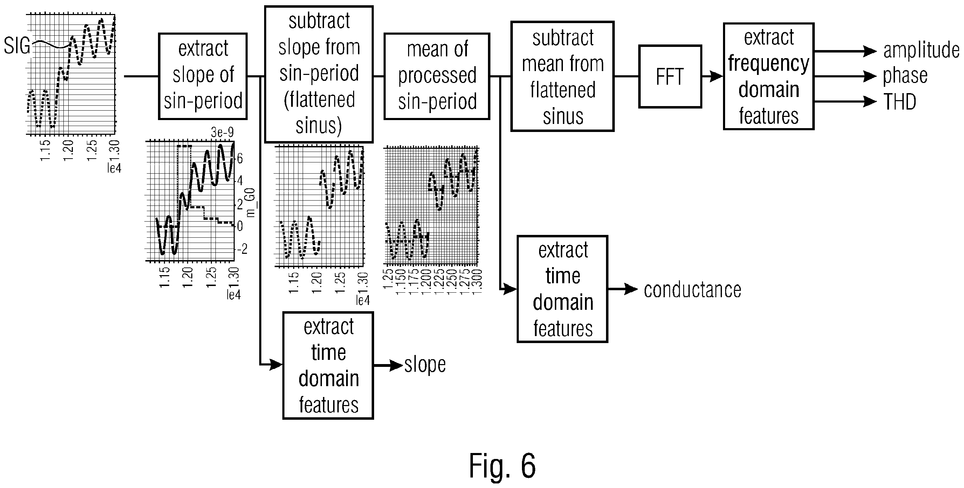

Figure 6 illustrates an exemplary implementation of the extraction of the feature values TFV, FFV for a (distorted) periodic signal SIG of the sensor 2, which is a sinusoidal signal SIG. The extraction of the feature values TFV, FFV is performed on single sine periods PER at a time. First, the slope is extracted, which may be used both directly as a time domain feature value TFV, representing the rate of change, as well as to flatten the waveform of the signal SIG. By subtracting the slope proportional to the length of the period PER, the individual sine periods PER are flattened, meaning the resulting sine periods PER are stationary. As a next step, the mean value of each period PER is calculated, which is the next time domain feature value TFV, representing the average conductance (or resistance) normalized to a baseline and is called sensitivity. As a final step in the time domain, the mean is subtracted from the periods PER of the signal SIG, removing the remaining DC offset of the periods PER of the signal SIG. The resulting periods PER of the signal SIG are then further processed in the frequency domain using a fast Fourier transform (FFT) algorithm in order to produce the spectrum of the respective period PER. The main features are the amplitude and the phase of the individual harmonics as well as the total harmonic distortion (THD). The calculation of the frequency domain feature values can be described as follows:

$$X[f] = FFT(x[n])$$

$$Amplitude[f] := |X[f]|$$

$$Phase[f] := \arg(X[f])$$

$$THD := \frac{\sqrt{\sum_{i=2}^{10} |X[i]|^2}}{|X[1]|}$$

The definition for the total harmonic distortion used here is the ratio of the square root of the quadratic sum of the amplitude of the higher order harmonics to the fundamental frequency. For practicality a limit for the harmonics considered can be set, e.g. 10 harmonic components, which should result in no noticeable loss of information.

If feature values TFV, FFV are extracted one period PER after the other, the length of the periods PER is effectively setting the output rate of the sensing results SR. To overcome this a sliding window with the length of the periods PER and overlap between periods PER can be applied, practically allowing any rate of features extraction over time necessary for the application. For example, shifting the sliding window by one minute, results in a new set of feature values TFV, FFV produced every minute independent of the length of the periods PER.

After the feature extraction processor 5, the feature values TVF, FFV are used as input to a trained model based algorithm processor 7 to make actual predictions on gas concentration levels. The recurrent neural network illustrated in Figure 1 has proven to be a sound choice for many scenarios, as it performs well on the time-series data provided by the gas sensor 2.

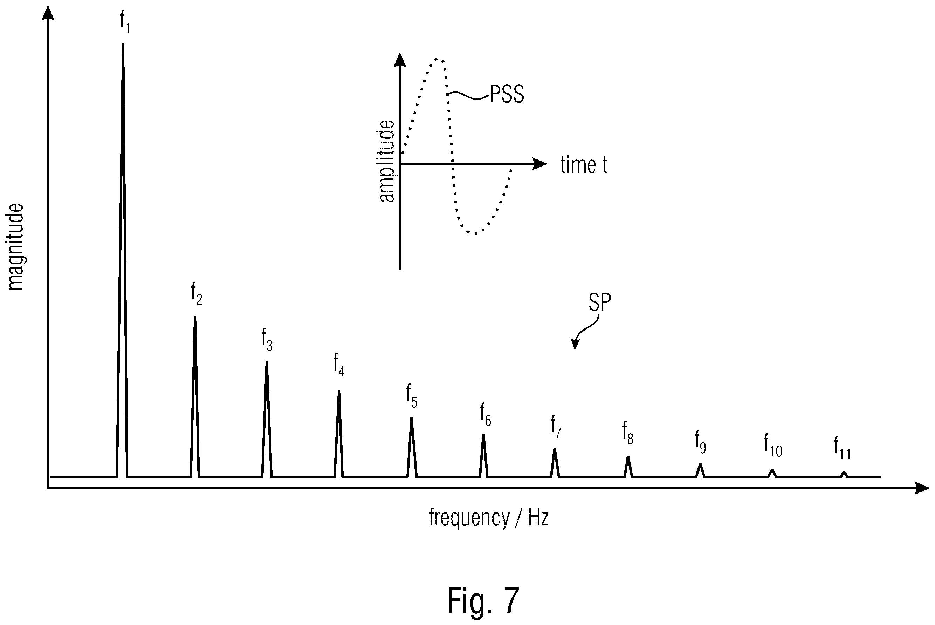

Figure 7 illustrates an exemplary preprocessed signal sample PPS and a spectrum SP derived therefrom. It can be seen, that the sinusoidal preprocessed signal sample PPS is clearly distorted. Moreover, it can be seen that the spectrum SP comprises a fundamental frequency $f_1$ and ten harmonics $f_2$ to $f_{11}$.

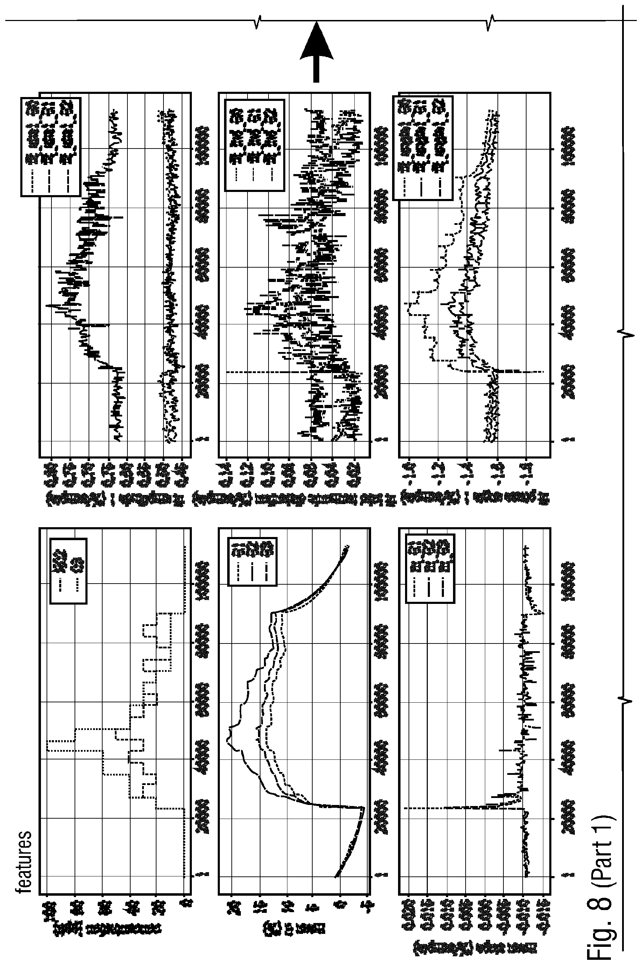

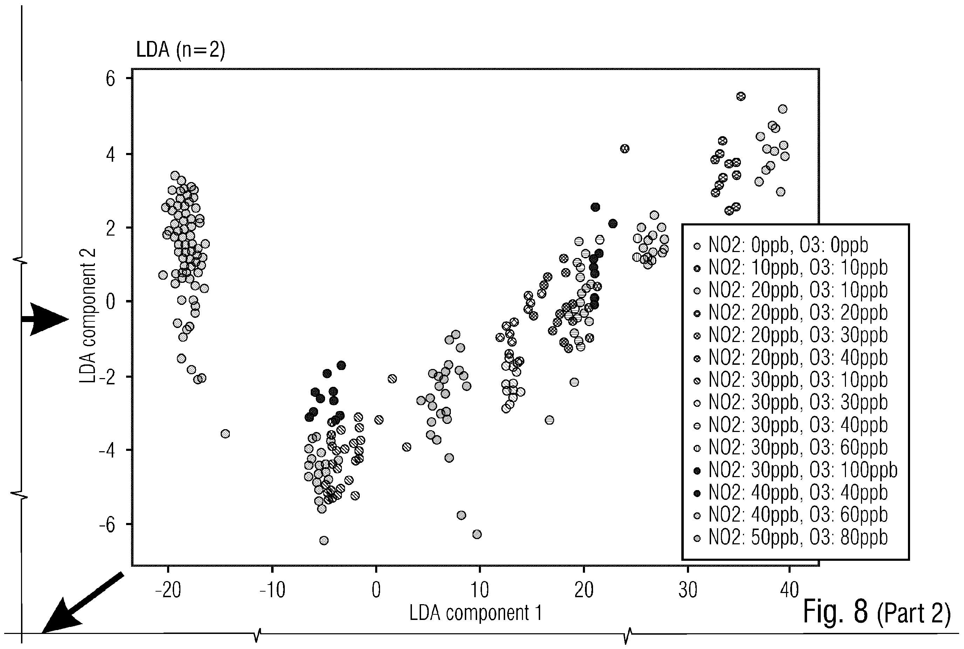

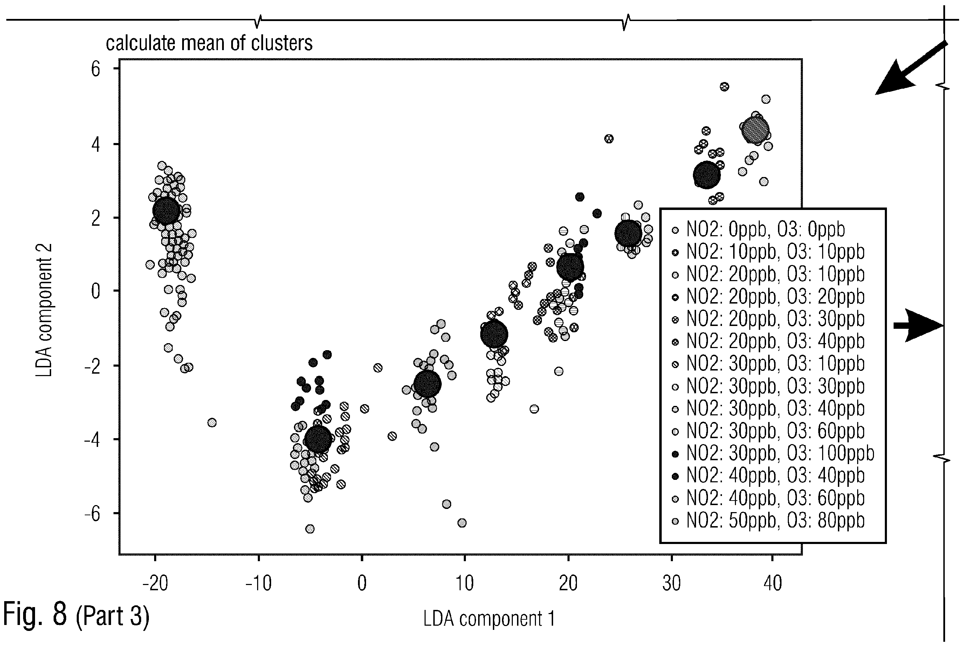

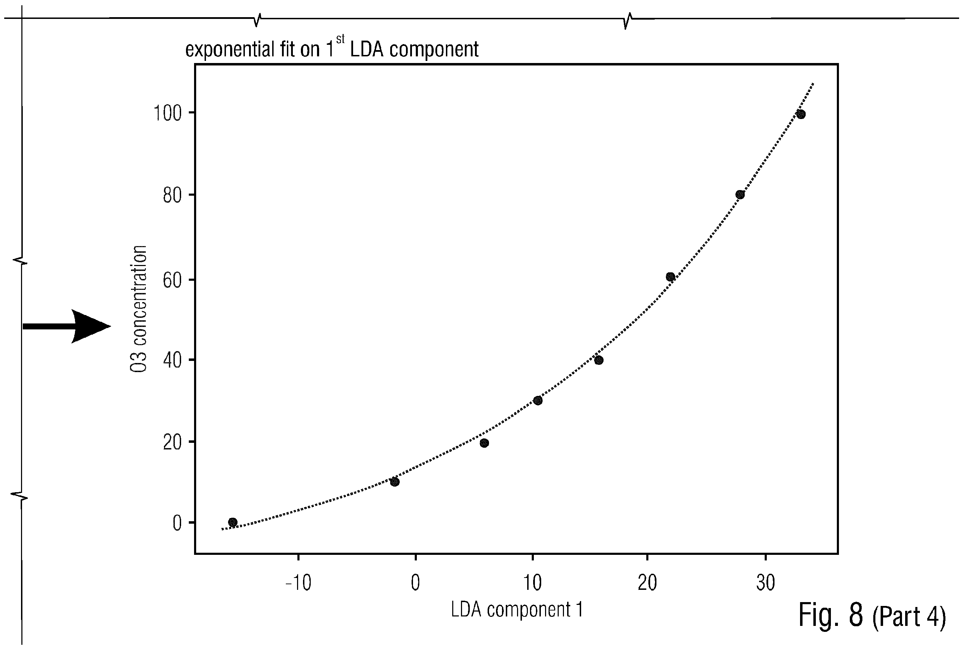

Figure 8 illustrates exemplary training steps for a trained model 8 using linear discriminant analysis.

If, for example, less data is available to train a neural network, other approaches such as a model based on linear discriminant analysis (LDA) for feature transformation and dimensionality reduction and a simple exponential or polynomial regression function are viable as well. The features based on the periodic heater mode work just as well with classical machine learning approaches as they do with neural networks.

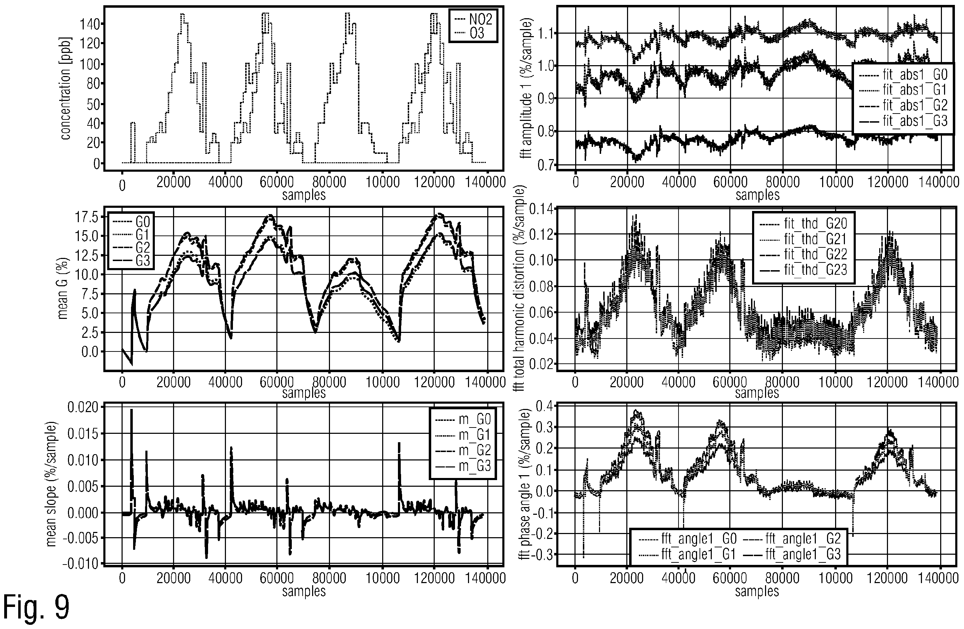

Figure 9 illustrates exemplary plots of exemplary time domain feature values TFV and of exemplary frequency domain feature values FFV for a specific gas concentration profile, which comprises nitrogen dioxide and ozone, and which is shown top-left. The mean normalized conductivities G0 to G3 of the four sensors 2.1 to 2.4 of Figure 5 are shown middle-left and the corresponding slopes m_G0 to m_G3 are shown bottom-left. The conductivities G0 to G3 and the slopes m_G0 to m_G3 may be used as time domain feature values TFV.

The corresponding amplitudes fft_abs1_G0 to fft_abs1_G3 of the fundamental frequency are shown top-right, the corresponding total harmonic distortions fft_thd_G20 to fft_thd_G23 are shown middle-right, and the corresponding phases fft_angle1_G0 to fft_angle1_G3 are shown bottom-right. The amplitudes fft_abs1_G0 to fft_abs1_G3, the total harmonic distortions fft_thd_G20 to fft_thd_G23, and the phases fft_angle1_G0 to fft_angle1_G3 may be used as frequency domain feature values.

It is visible that especially the total harmonic distortions fft_thd_G20 to fft_thd_G23 and the phases fft_angle1_G0 to fft_angle1_G3 of the fundamental frequency show a strong correlation to the ozone concentration. They are however barely responsive to nitrogen dioxide which is due to the specific mode of operation (temperature, period length). When comparing the total harmonic distortions fft_thd_G20 to fft_thd_G23 and the phases fft_angle1_G0 to fft_angle1_G3 with the mean normalized conductivities G0 to G3, it is visible that the frequency domain feature values offer a much faster response and return quickly to the initial value when the ozone concentration goes to zero. The mean normalized conductivities G0 to G3 on the other hand are susceptible to slow response and drift, which makes it less robust on its own.

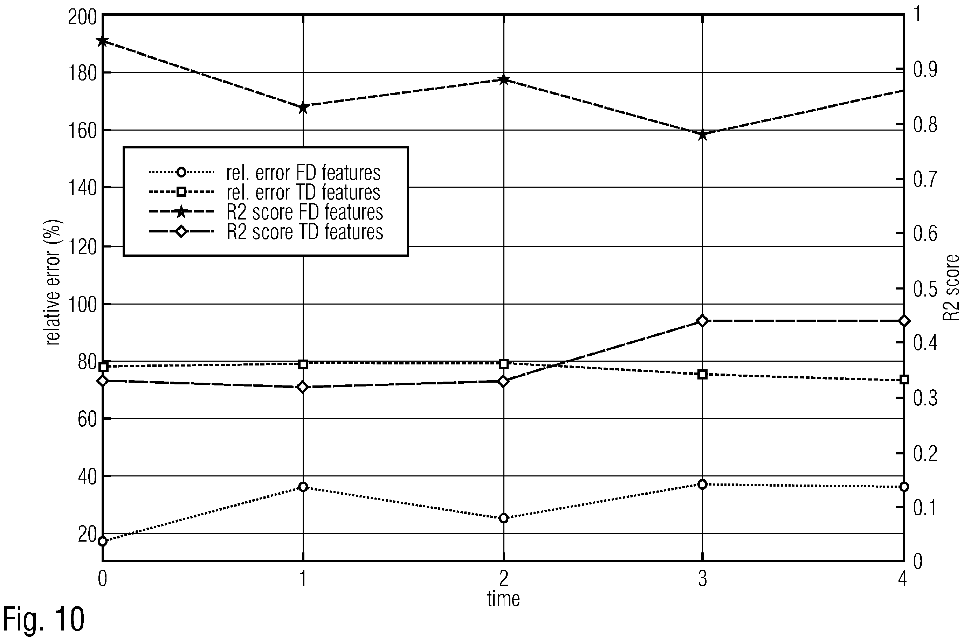

Figure 10 illustrates an exemplary plot of an exemplary relative estimation error and of an exemplary R2 score for time domain feature values TFV and for frequency domain feature values FFV as a function of time. Figure 10 shows the performance improvement obtained in terms of relative error ("Rel. Error FD Features") and R2 score ("R2 score FD Features") when using the newly extracted frequency domain feature values FFV versus the relative error ("Rel. Error TD Features") and the and R2 score ("R2 score TD Features") of the time domain feature values TFV in the presence of drifting signals SIG in the field. Time domain feature values TFV include the slope and sensitivity illustrated also in Figure 4 and extracted prior to the time-to-frequency processor 15.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

The above described is merely illustrative, and it is understood that modifications and variations of the arrangements and the details described herein will be apparent to others skilled in the art. It is the intent, therefore, to be limited only by the scope of the impending claims and not by the specific details presented by way of description and explanation above.

References:

[1] A. Vergara , E. Martinelli, E. Llobet, A. D'Amico, C. Di Natale, "Optimized Feature Extraction for Temperature-Modulated Gas Sensors", Journal of Sensors Volume 2009, Article ID 716316.

[2] Alexey Lipatov, Alexey Varezhnikov, Peter Wilson, Victor Sysoev, Andrei Kolmakov and Alexander Sinitskii, "Highly selective gas sensor arrays based on thermally reduced graphene oxide", Nanoscale, 2013, 5, 5426-5434.

[3] M. Baumbach et al.:"A new method for fast identification of gases and gas mixtures after sensor power up", IEEE Sensors 2004, Vienna, Austria, Oct. 25-27, 2004.

[4] Oriol Gonzalez, et al., "A Novel Modular eNose System Based on Commercial MOX Sensors to Detect Low Concentrations of VOCs for Breath Gas Analysis ", Proceedings of Eurosensors 2018, Graz, September 2018.

[5] Romana Boiger et al., "Exploring Temperature-Modulated Operation Mode of Metal Oxide Gas Sensors for Robust Signal Processing", Proceedings of Eurosensors 2018, Graz, September 2018.

**Claims**

1. A gas sensing device for sensing one or more gases in a mixture of gases; the gas sensing device (1) comprising:

   one or more chemo-resistive gas sensors (2), wherein each of the gas sensors (2) is configured for generating signals (SIG) corresponding to concentrations of the one or more gases in the mixture of gases;
   a heating arrangement (3) configured in such way that the gas sensors (2) are heated according to a periodic temperature profile (STP);
   a preprocessing processor (4) configured for receiving the signals (SIG) from each of the gas sensors (2) and for preprocessing the received signals (SIG) in order to generate a preprocessed signal sample (PSS) for each of the gas sensors (2) for each period (PER) of the periodic temperature profile (STP);
a feature extraction processor (5) configured for receiving the preprocessed signal samples (PSS) and for extracting for each of the periods (PER) a set of feature values (TFV, FFV) from the preprocessed signal samples (PSS) received for the respective period (PER), wherein each of the sets of feature values (TFV, FFV) comprises one or more time domain feature values (TFV), which are based on time domain characteristics of one of the preprocessed signal samples (PSS) received for the respective period (PER), and one or more frequency domain feature values (FFV), which are based on frequency domain characteristics of one of the preprocessed signal samples (PSS) received for the respective period (PER);
a gas concentration processor (6) configured for receiving the sets of feature values (TFV, FFV), and for creating for each of the sets of feature values (TFV, FFV) a sensing result (SR) for each of the gases, wherein the gas concentration processor (6) comprises a trained model based algorithm processor (7) and one or more trained models (8) for the trained model based algorithm processor (7), wherein at least a portion of the respective set of feature values (TFV, FFV) is fed to inputs (9) of the trained model based algorithm processor (7) using one of the one or more trained models (8), wherein the sensing results (SR) are based on output values (OV) at outputs (10) of the trained model based algorithm processor (7).

2. A gas sensing device according to the preceding claim, wherein the preprocessing processor (4) comprises a baseline normalization processor (11) configured for normalizing the signals (SIG) received from the gas sensors (2).

3. A gas sensing device according to one of the preceding claims, wherein the preprocessing processor (4) comprises a sine period extraction processor (12) configured for receiving a control signal (CS) from the heating arrangement (3) and for extracting a starting time (ST) and an end time (ET) for one of the periods (PER) from the control signal (CS).

4. A gas sensing device according to one of the preceding claims, wherein the feature extraction processor (5) comprises a slope calculation processor (13) configured for calculating for each of the periods (PER) a slope for each of the preprocessed signal samples (PSS) received for the respective period (PER), wherein the feature extraction processor (5) is configured for using the slope of one of the periods (PER) as one of the time domain feature values (TFV) of the set of feature values (TFV, FFV) of the respective period (PER).

5. A gas sensing device according to one of the preceding claims, wherein the feature extraction processor (5) comprises a sensitivity calculation processor (14) configured for calculating for each of the periods (PER) a sensitivity for each of the preprocessed signal samples (PSS) received for the respective period (PER), wherein the feature extraction processor (5) is configured for using the sensitivity of one of the periods (PER) as one of the time domain feature values (TFV) of the set of feature values (TFV, FFV) of the respective period (PER).

6. A gas sensing device according to one of the preceding claims, wherein the feature extraction processor (5) comprises a time-to-frequency converting processor (15) configured for converting each of the preprocessed signal samples (PSS) from a time domain into a frequency domain in order to calculate a spectrum (SP) for each of the preprocessed signal samples (PSS), wherein the frequency domain feature values (FFV) for the respective preprocessed signal sample (PSS) are calculated from the respective spectrum (SP).

7. A gas sensing device according to claim 6, wherein the feature extraction processor (5) comprises an amplitude calculation processor (16) configured for calculating for each of the periods (PER) at least one amplitude of the spectrum (SP) of each of the preprocessed signal samples (PSS) received for the respective period (PER), wherein the feature extraction processor (5) is configured for using at least one of the amplitudes calculated for one of the periods (PER) as one of the frequency domain feature values (FFV) of the set of feature values (TFV, FFV) of the respective period (PER).

8. A gas sensing device according to claim 6, wherein the feature extraction processor (5) comprises a phase calculation processor (17) configured for calculating for each of the periods (PER) at least one phase of the spectrum (SP) of each of the preprocessed signal samples (PSS) received for the respective period (PER), wherein the feature extraction processor (5) is configured for using at least one of the phases calculated for one of the periods (PER) as one of the frequency domain feature values (FFV) of the set of feature values (TFV, FFV) of the respective period (PER).

9. A gas sensing device according to claim 6, wherein the feature extraction processor (5) comprises a total harmonic distortion calculation processor (18) configured for calculating for each of the periods (PER) at least one total harmonic distortion of the spectrum (SP) of each of the preprocessed signal samples

(PSS) received for the respective period (PER), wherein the feature extraction processor (5) is configured for using at least one of the total harmonic distortions calculated for one of the periods (PER) as one of the frequency domain feature values (FFV) of the set of feature values (TFV, FFV) of the respective period (PER).

10. A gas sensing device according to one of the preceding claims, wherein the preprocessing processor (4) comprises a relative resistance extracting processor (19) configured for extracting relative resistance changes of the gas sensors (2) from the signals (SIG) received from the gas sensors (2), wherein the preprocessing processor (4) is configured for using the relative resistance changes for generating the preprocessed signal samples (PSS).

11. A gas sensing device according to one of the preceding claims, wherein the preprocessing processor (4) comprises a relative conductance extracting processor (20) configured for extracting relative conductance changes of the gas sensors (2) from the signals (SIG) received from the gas sensors (2), wherein the preprocessing processor (4) is configured for using the relative conductance changes for generating the preprocessed signal samples (PSS).

12. A gas sensing device according to one of the preceding claims, wherein the gas sensing device (2) comprises

a drift calculation processor (21) configured for calculating for each of the sensors (2) a drift of the respective sensor signal (SIG), and
a feature selection processor (22) configured for selecting for each of the sensors (2), based on the drift of the respective sensor (2), which of the feature values (TFV, FFV) of one of the sets of feature values (TFV, FFV) are fed to the inputs (9) of the trained model based algorithm processor (7).

13. A gas sensing device according to one of the preceding claims, wherein the periodic temperature profile (STP) is a sinusoidal temperature profile (STP).

14. A method for operating a gas sensing device (1) for sensing one or more gases in a mixture of gases; the gas sensing device (1) comprising one or more chemo-resistive gas sensors (2), wherein the method comprises the steps of::

using each of the gas sensors (2) for generating signals (SIG) corresponding to concentrations of the one or more gases in the mixture of gases; using a heating arrangement (3) in such way that the gas sensors (2) are heated according to a periodic temperature profile (STP); using a preprocessing processor (4) for receiving the signals (SIG) from each of the gas sensors (2) and for preprocessing the received signals (SIG) in order to generate a preprocessed signal sample (PSS) for each of the gas sensors (2) for each period (PER) of the periodic temperature profile (STP); using a feature extraction processor (5) for receiving the preprocessed signal samples (PSS) and for extracting for each of the periods (PER) a set of feature values (TFV, FFV) from the preprocessed signal samples (PSS) received for the respective period (PER), wherein each of the sets of feature values (TFV, FFV) comprises one or more time domain feature values (TFV), which are based on time domain characteristics of one of the preprocessed signal samples (PSS) received for the respective period (PER), and one or more frequency domain feature values (FFV), which are based on frequency domain characteristics of one of the preprocessed signal samples (PSS) received for the respective period (PER); using a gas concentration processor (6) for receiving the sets of feature values (TFV, FFV), and for creating for each of the sets of feature values (TFV, FFV) a sensing result (SR) for each of the gases, wherein the gas concentration processor (6) comprises a trained model based algorithm processor (7) and at least one trained model (8) for the trained model based algorithm processor (7), wherein at least a portion of the respective set of feature values (TFV, FFV) is fed to inputs (9) of the trained model based algorithm processor (7), wherein the sensing results (SR) are based on output values (OV) at outputs (10) of the trained model based algorithm processor (7).

Fig. 1

Fig. 2

EP 4 246 137 A1

Fig. 3

Fig. 4

Fig. 5

EP 4 246 137 A1

Fig. 6

Fig. 7

EP 4 246 137 A1

features

Fig. 8 (Part 1)

Fig. 8 (Part 2)

Fig. 8 (Part 3)

exponential fit on 1st LDA component

Fig. 8 (Part 4)

EP 4 246 137 A1

Fig. 9

Fig. 10

EP 4 246 137 A1

Europäisches
Patentamt

European
Patent Office

Office européen
des brevets

# EUROPEAN SEARCH REPORT

Application Number

EP 22 16 1857

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | IONESCU R ET AL: "Ethanol and H"2S gas detection in air and in reducing and oxidising ambience: application of pattern recognition to analyse the output from temperature-modulated nanoparticulate WO"3 gas sensors", SENSORS AND ACTUATORS B: CHEMICAL, ELSEVIER BV, NL, vol. 104, no. 1, 3 January 2005 (2005-01-03), pages 124-131, XP027810261, ISSN: 0925-4005 [retrieved on 2005-01-03] | 1-11,13, 14 | INV. G01N27/12 G01N33/00 |
| A | * pages 125,128 * | 12 | |
| A | EP 3 936 861 A1 (INFINEON TECHNOLOGIES AG [DE]) 12 January 2022 (2022-01-12) * the whole document * | 1-14 | |
| A | AIXIANG HE ET AL: "Short-Time Fourier Transform and Decision Tree-Based Pattern Recognition for Gas Identification Using Temperature Modulated Microhotplate Gas Sensors", JOURNAL OF SENSORS, vol. 2016, 1 January 2016 (2016-01-01), pages 1-12, XP055745561, US ISSN: 1687-725X, DOI: 10.1155/2016/7603931 * the whole document * | 1-14 | TECHNICAL FIELDS SEARCHED (IPC) G01N |

The present search report has been drawn up for all claims

| Place of search | Date of completion of the search | Examiner |
|---|---|---|
| Munich | 20 July 2022 | Klein, Marc-Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 1857

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2022

| Patent document cited in search report | | Publication date | Patent family member(s) | | Publication date |
|---|---|---|---|---|---|
| EP 3936861 | A1 | 12-01-2022 | CN 113988146 | A | 28-01-2022 |
| | | | EP 3936861 | A1 | 12-01-2022 |
| | | | KR 20220007538 | A | 18-01-2022 |
| | | | US 2022011283 | A1 | 13-01-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. VERGARA ; E. MARTINELLI ; E. LLOBET ; A. D'AMICO ; C. DI NATALE.** Optimized Feature Extraction for Temperature-Modulated Gas Sensors. *Journal of Sensors,* vol. 2009
- **ALEXEY LIPATOV ; ALEXEY VAREZHNIKOV ; PETER WILSON ; VICTOR SYSOEV ; ANDREI KOLMAKOV ; ALEXANDER SINITSKII.** Highly selective gas sensor arrays based on thermally reduced graphene oxide. *Nanoscale,* 2013, vol. 5, 5426-5434
- **M. BAUMBACH et al.** A new method for fast identification of gases and gas mixtures after sensor power up. *IEEE Sensors,* 2004
- **ORIOL GONZALEZ et al.** A Novel Modular eNose System Based on Commercial MOX Sensors to Detect Low Concentrations of VOCs for Breath Gas Analysis. *Proceedings of Eurosensors 2018, Graz,* September 2018
- **ROMANA BOIGER et al.** Exploring Temperature-Modulated Operation Mode of Metal Oxide Gas Sensors for Robust Signal Processing. *Proceedings of Eurosensors 2018, Graz,* September 2018